# EUROPEAN PATENT SPECIFICATION

(11) **EP 2 116 113 B1**
(45) Date of publication and mention of the grant of the patent: **26.03.2014**
(21) Application number: 08712465.7
(22) Date of filing: 12.02.2008
(51) Int. Cl.: H01J 37/16, H05H 1/34, A61L 2/14, H01J 37/32

(54) **DOOR FOR VACUUM CHAMBER**
TÜR FÜR EINE VAKUUMKAMMER
PORTE POUR CHAMBRE A VIDE

(30) Priority: 12.02.2007 KR 20070014230
(43) Date of publication of application: 11.11.2009
(73) Proprietor: Human Meditek Co., Ltd., Seoul 150-023 (KR)
(72) Inventor: KO, Jung Suek, Seoul 135-270 (KR)
(74) Representative: Maser, Jochen
(86) International application number: PCT/KR2008/000818
(87) International publication number: WO 2008/100056

(56) References cited:
- EP-A1- 0 019 293
- WO-A1-99/03135
- JP-A- 09 199 293
- JP-A- 2002 266 546
- SU-A1- 1 093 854
- US-A- 4 044 918
- US-A- 5 007 183
- US-A- 5 313 738
- US-B2- 6 932 354

## Description

### [Technical Field]

The present invention relates, in general, to a door for a vacuum chamber and, more particularly, to a door for a vacuum chamber, which is used to put an article into the chamber, and is constructed to prevent the door from being deformed by a bending load when a vacuum is created in the vacuum chamber.

### [Background Art]

As is well known to those skilled in the art, methods of using plasma, which is the fourth state of matter, and is distinct from three states of matter, namely, solid, liquid, and gas, have been widely adopted in all industrial fields.

For example, in order to manufacture semiconductors and flat panel displays as well as fluorescent lights and neon lights, which are widely used in daily life, several manufacturing processes are required. Among the manufacturing processes, the process of manufacturing a wafer or substrate includes unit steps, such as a dry etching step, a physical or chemical vapor deposition step, a photoresist washing step, etc. In these unit steps, plasma is widely used.

Further, the method of using plasma is used to disinfect or sterilize articles on which the presence of germs is unacceptable, such as medical instruments.

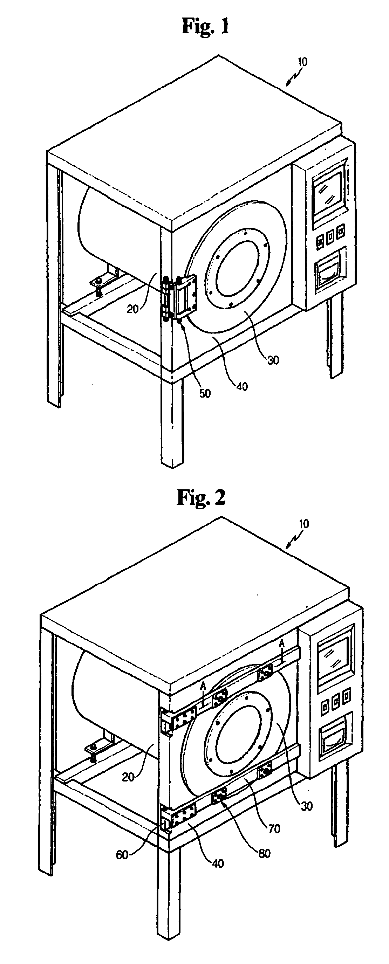

The applicant of the present invention has been granted many patents for a plasma sterilizer, which sterilizes an article, such as a medical instrument, using hydrogen peroxide, and decomposes the used hydrogen peroxide into nontoxic matter using plasma, prior to discharging the nontoxic matter outside the sterilizer. As shown in FIG. 1, a plasma sterilizer 10 is provided with a vacuum chamber 20, in which a vacuum is formed. A door 30 is mounted to one surface of the chamber 20, and is used for insertion of an article to be sterilized, such as a medical instrument, into the chamber.

Only one surface of the chamber 20 is open, and the remainder of the chamber is hermetically sealed. The door 30 is mounted to the open surface of the chamber so as to open or close it.

A base plate 40, which is open at a central portion thereof, is mounted to the opening of the chamber 20. The door 30 is provided on the front of the base plate 40 so as to open or close the central opening of the base plate. The door 30 is coupled at one end thereof to one end of the base plate 40 via a hinge assembly 50. Thus, as the door 30 rotates around the hinge assembly 50 relative to the base plate 40, the chamber is opened or closed.

The door 30 rotates around the hinge assembly 50, thus opening the opening of the base plate 40 and the opening which is provided on one surface of the chamber 20. The article to be sterilized, such as the medical instrument, is put into the chamber through the openings. Subsequently, the door 30 rotates around the hinge assembly 50 again, thus closing the opening of the base plate 40 and the opening of the chamber 20.

In this state, plasma is generated in the chamber 20, so that the article is sterilized.

In this case, in order to sterilize the article using the plasma generated in the chamber 20, a vacuum must be created in the chamber 20. As the vacuum is created in the chamber 20, the door 30 is pulled towards the chamber 20 by external atmospheric pressure.

As such, if the door 30 is repeatedly pulled towards the chamber 20 each time the chamber 20 is evacuated, the hinge assembly 50 of the door 30 may be deformed due to the load. Thereby, the entire door 30 may be distorted.

That is, the hinge assembly 50 of the door 30 is mounted to only one end of the door 30. Thus, as the door 30 is pulled inwards relative to the chamber 20 due to the vacuum in the chamber 20, stress concentrates on the hinge assembly 50. Hence, when the hinge assembly 50 is deformed due to a load, and thus the door 30 is distorted, the door 30 cannot perfectly close the chamber 20. As a result, it is difficult to create a vacuum in the chamber 20.

Therefore, the door 30 must be frequently replaced with a new one, so that the maintenance cost is increased. The US 4,044,918 discloses a high temperature, quick access door for high vacuum chambers, which comprises a chamber having in one surface thereof an opening. A sealing disc is provided on a supporting arm for rotatably opening or closing the opening of the chamber. This supporting arm is fastened to an outer surface of the door in such a way as to be spaced apart from the door by a predetermined interval and the support arm is rotatable coupled at one end thereof to a hinge assembly. This sealing disc will be manually operated for closing and opening by a thrust bolt, whereby a path of movement of the sealing disc is along the axis of the sealing disc.

### (Disclosure)

### (Technical Problem)

Accordingly, the present invention has been made keeping in mind the above problems occurring in the prior art, and an object of the present invention is to provide a door for a vacuum chamber, which is constructed to prevent the door, for opening or closing an opening formed in the one surface of the chamber, from being deformed by a bending load generated during the evacuation of the chamber, thus reliably creating a vacuum in the chamber, and reducing the cost of maintenance of the door.

### (Technical Solution)

In order to accomplish the above object, the present invention provides a door for a vacuum chamber, including a chamber having in one surface thereof an opening; a door rotated to open or close the opening of the chamber; and a guide plate which is fastened to an outer surface of the door in such a way as to be spaced apart from the door by a predetermined interval, and is rotatably coupled at one end thereof to a hinge assembly.

Further, first grooves are formed at regular intervals in a surface of the door contacting the guide plate, and second grooves are formed in the guide plate to correspond to the first grooves in the door, so that, when the guide plate contacts the door, a space having a predetermined size is defined between the first and second grooves. Additionally, an elastic member is installed in the space, is secured at a first end thereof to an inner surface of each of the first grooves in the door, and is secured at a second end thereof to an inner surface of each of the second grooves in the guide plate.

Further, a block is fastened to the guide plate, increases strength of the guide plate, and has a hole for guiding the guide bushing.

The door includes fastening holes preferably having a predetermined depth at regular intervals, and the guide plate includes through holes corresponding to the fastening holes, so that a fastening member passes through a corresponding through hole and is secured at an end thereof to the corresponding fastening hole, thus fastening the guide plate to the door.

Each of the first grooves in the door is preferably formed around a corresponding fastening hole to have a predetermined depth, and each of the second grooves in the guide plate is formed around a corresponding through hole to have a predetermined depth.

Preferably, the elastic member comprises a coil spring.

Preferably, the door further includes a guide bushing having a hole through which the fastening member passes. The guide bushing includes a first part which passes through a corresponding through hole in the guide plate and slides a predetermined interval in the space, and a second part which has a diameter larger than the through hole in the guide plate and is stopped by an outer surface of the guide plate.

When the end of the fastening member is completely fastened to each of the first grooves in the door, a predetermined gap is preferably defined between the second part of the guide bushing and the outer surface of the guide plate.

A base plate, having a central portion thereof an opening, is preferably provided on a front of the opening of the chamber, and the door is rotated to open or close the opening of the base plate and the opening of the chamber.

In particular, the hinge assembly is mounted to an end of the base plate, and an end of the guide plate is rotatably coupled to the hinge assembly.

### (Advantageous Effects)

As described above, the present invention provides a door for a vacuum chamber, which rotates around a hinge assembly provided on one side of the door, and which is constructed so that guide plates are provided on the upper and lower portions of the door in such a way as to be spaced apart from the door by a predetermined interval, and each of the guide plate is rotably coupled at one end thereof to the fixed hinge assembly, and an elastic member is interposed between the door and the corresponding guide plate, thus allowing the guide plate to remain in a fixed position, even if the door is pulled towards the chamber when the vacuum chamber is evacuated, therefore preventing stress from being concentrated on a part of the door coupled to the hinge assembly, and preventing the hinge assembly from being deformed.

In a detailed description, since the part of the door coupled to the hinge assembly is not deformed due to the concentration of stress, the door always reliably closes the opening of the chamber. Thus, plasma treatment can be smoothly conducted because the vacuum is reliably formed.

Further, continuous maintenance for the door is not required, thus reducing maintenance costs.

### [Description of Drawings]

FIG. 1 is a perspective view showing a conventional plasma sterilizer, and showing the coupling of a vacuum chamber with a door;
FIG. 2 is a perspective view showing the coupling of a vacuum chamber with a door, according to the present invention;
FIG. 3 is a sectional view taken along line A-A of FIG. 2; and
FIG. 4 is an exploded sectional view of FIG. 3.

**<Description of reference characters of important parts>**

| | | | |
|---|---|---|---|
| 10: | plasma sterilizer | | |
| 20: | chamber | 30: | door |
| 32: | groove | 34: | fastening hole |
| 40: | base plate | 60: | hinge assembly |
| 70: | guide plate | 72: | groove |
| 74: | through hole | 82: | fastening member |
| 84: | guide bushing | 84a: | hole |
| 84b: | first part | 84c: | second part |
| 90: | elastic member | 92: | space |

### [Best Mode]

Hereinafter, the preferred embodiment of the present invention will be described in detail with reference to the accompanying drawings.

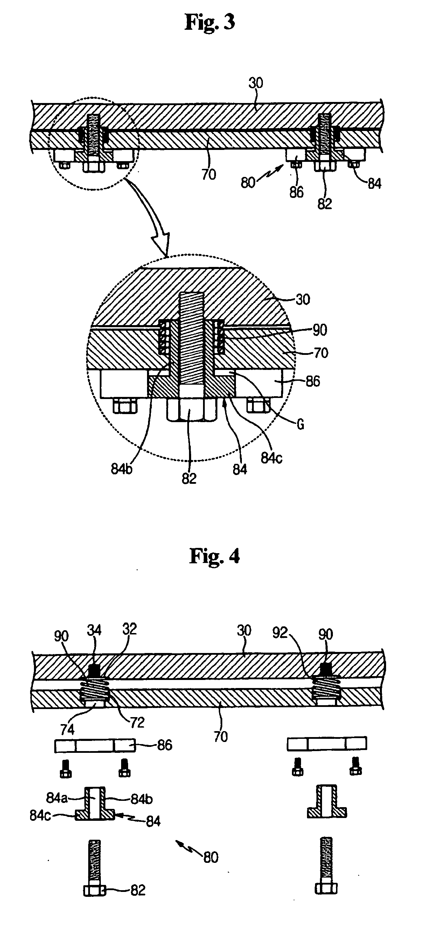

FIG. 2 is a perspective view showing the coupling structure of a door for a vacuum chamber, according to the present invention, FIG. 3 is a sectional view taken along line A-A of FIG. 2, and FIG. 4 is an exploded sectional view of FIG. 3.

As shown in the drawings, a plasma sterilizer is provided with a chamber 20 which creates a vacuum so as to generate plasma, and a door 30 which is mounted to one surface of the chamber 20 and is used to put articles to be sterilized, for example, medical instruments, into the chamber 20.

The chamber 20 is hermetically sealed, except for an opening which is provided in one surface of the chamber. The door 30 is mounted so as to open or close the opening.

In a detailed description, a base plate 40, which is open at a central portion thereof, is mounted to the opening of the chamber 20. The door 30 is provided on the front of the base plate 40 so as to open or close the opening which is formed in the central portion of the base plate 40.

The base plate 40 is part of a frame for supporting the chamber 20, and is secured to the chamber. Guide plates 70 are rotatably mounted to one surface of the base plate 40, and are fastened to the door 30.

That is, hinge assemblies 60 are provided on one end of the base plate 40.- Each guide plate 70 is rotatably coupled at one end thereof to the corresponding hinge assembly 60. The guide plate 70 is fastened to the door via a plurality of fastening units 80.

Meanwhile, grooves 32, each having a predetermined depth, are provided at regular intervals in the outer surface of the door 30 which contacts each guide plate 70. A fastening hole 34 is formed in the center of each groove 32 and is deeper than the groove.

Further, grooves 72, each having a predetermined depth, are provided on the inner surface of each guide plate 70 which contacts the outer surface of the door 30, and correspond to the grooves 32 in the door 30.

Thus, when each guide plate 70 contacts the door 30, the grooves 32 and 72 overlap each other, thus defining a space 92 having a predetermined size.

Further, a through hole 74 is formed in the center of each groove 72 in each guide plate 70, so that a guide bushing 84 and a fastening member 82, which will be described below, pass through the through hole.

The guide bushing 84 has a "T"-shaped section, and includes a first part 84b and a second part 84c. The first part passes through the corresponding through hole 74 in each guide plate 70, and slides a predetermined interval in the space 92. The second part has a larger diameter than the through hole 74 in the guide plate 70, so that the second part is stopped by the outer surface of the guide plate 70.

A hole 84a is formed in the guide bushing 84 so that the fastening member 82 passes through the guide bushing. Thus, after the fastening member 82 is inserted into the hole 84a in the guide bushing 84, the end of the fastening member 82 is fastened to the corresponding fastening hole 34 of the door 30. When the end of the fastening member 82 is completely fastened to the corresponding fastening hole 34 of the door 30, the second part 84c of the guide bushing 84 is spaced apart from the outer surface of the guide plate 70 by a predetermined interval, thus defining a gap G between the outer surface of the guide plate and the second part. The reason why this gap is formed will be described below.

Further, an elastic member 90 is installed in each space 92, which is defined between the grooves 32 and 72 in the door 30 and each guide plate 70, and surrounds the outer surface of the first part 84b of the guide bushing 84. One end of the elastic member 90 is supported by the inner surface of each groove 32 in the door 30, while the other end of the elastic member is supported by the inner surface of the corresponding groove 72 in each guide plate 70.

Any member may be used as the elastic member 90, as long as the member has predetermined elasticity. It is preferable that a coil spring be used as the elastic member.

Further, blocks 86 are mounted to each guide plate 70 via bolts. Each block surrounds the corresponding guide bushing 84 so as to guide the sliding motion of the guide bushing, and increases the strength of the guide plate 70.

The operation of the door for the vacuum chamber, which is constructed as described above, will be described below.

First, the door 30 is opened to put an article to be sterilized into the chamber 20. In this state, the door 30 is closed again so as to seal the chamber 20. Thereafter, a vacuum is created in the chamber 20, thus generating plasma.

At this time, each guide plate 70, which is fastened to the door 30 via the fastening units 80, rotates around the corresponding hinge assembly 60, which is mounted to one end of the base plate 40. As the guide plate rotates, the door may be opened or closed.

When a vacuum is created in the chamber 20, the door 30 is pulled towards the chamber 20 by external atmospheric pressure.

The fastening members 82 are fastened to the corresponding fastening holes 34, which are formed in the upper and lower edges of the door 30. As the door 30 is pulled, the fastening members 82 move inwards.

At this time, each elastic member 90, which is installed in the space 92 defined between each of the grooves 32 in the door 30 and the corresponding groove 72 in each guide plate 70, is extended as the door 30 is pulled. Simultaneously, each guide bushing 84, through which the fastening member 82 passes, is also pulled inwards by a predetermined interval.

As described above, the maximum interval by which each guide bushing 84 is pulled is equal to the gap G which is defined between the guide plate 70 and the second part 84c of the guide bushing 84.

That is, when the door 30 is pulled towards the chamber 20 by the vacuum pressure in the chamber 20, the fastening members 82 are also pulled inwards. At this time, each elastic member 90 is extended, and each guide bushing 84, through which the fastening member 82 passes, is moved inwards. Thereby, each guide plate 70 remains in a fixed position.

Thus, a large load is not applied to each hinge assembly 60, which is coupled to the corresponding guide plate 70 and the base plate 40.

Further, when the vacuum in the chamber 20 is released, so that the chamber 20 has atmospheric pressure, the door 30 is pushed outwards by the elastic restoring force of each elastic member 90, and is in close contact with each guide plate 70. Simultaneously, each fastening member 82 is pushed outwards along with the corresponding guide bushing 84. Thus, the second part 84c of each guide bushing 84 is spaced apart from the corresponding guide plate 70 by a predetermined interval.

While the invention has been described with reference to a vacuum chamber for a plasma sterilizer, as an example, the present invention may be applied to any vacuum chamber constructed such that a door is mounted to one surface of the vacuum chamber so as to open or close it.

## Claims

1. A door for a vacuum chamber,
the chamber (10) having in one surface thereof an opening;
the door (30) being adapted to rotate to open or close the opening of the chamber; and comprising :
a guide plate (70) fastened to an outer surface of the door (30) in such a way as to be spaced apart from the door (30) by a predetermined interval, the guide plate (70) being rotatably coupled at one end thereof to a hinge assembly (60), **characterised in that** first grooves (32) are formed at regular intervals in a surface of the door (30) contacting the guide plate (70), and second grooves (72) are formed in the guide plate (70) to correspond to the first grooves (32) in the door (30), so that, when the guide plate (70) contacts the door (30), a space (92) having a predetermined size is defined between the first and second grooves (32, 72),
an elastic member (90) is installed in the space (92), is secured at a first end thereof to an inner surface of each of the first grooves (32) in the door (30), and is secured at a second end thereof to an inner surface of each of the second grooves (72) in the guide plate (70), and
a block (86) is fastened to the guide plate (70), the block 86 is adapted to increase the strength of the guide plate (70), and has a hole for guiding a guide bushing (84).

2. The door according to claim 1, wherein the door (30) comprises fastening holes (34) having a predetermined depth at regular intervals, and the guide plate (70) comprises through holes (74) corresponding to the fastening holes (34), so that a fastening member (82) passes through a corresponding through hole (74) and is secured at an end thereof to the corresponding fastening hole (34), thus fastening the guide plate (70) to the door.

3. The door according to claim 2, wherein each of the first grooves (32) in the door (30) is formed around a corresponding fastening hole to have a predetermined depth, and each of the second grooves (72) in the guide plate (70) is formed around a corresponding through hole to have a predetermined depth.

4. The door according to claim 1, wherein the elastic member (90) comprises a coil spring.

5. The door according to claim 2, further comprising:
a guide bushing (84) having a hole through which the fastening member (82) passes, and comprising:
a first part passing through a corresponding through hole in the guide plate (70), and sliding a predetermined interval in the space (92); and
a second part having a diameter larger than the through hole in the guide plate, and stopped by an outer surface of the guide plate (70).

6. The door according to claim 5, wherein, when the end of the fastening member (82) is completely fastened to each of the first grooves (32) in the door, a predetermined gap is defined between the second part of the guide bushing (84) and the outer surface of the guide plate (70).

7. The door according to claim 1, wherein a base plate (40), having in a central portion thereof an opening, is provided on a front of the opening of the chamber (20).

8. The door according to claim 7, wherein the hinge assembly (60) is mounted to an end of the base plate (40), and an end of the guide plate (70) is rotatably coupled to the hinge assembly (60).

## Patentansprüche

1. Tür für eine Vakuumkammer,
wobei die Kammer (10) in einer Oberfläche eine Öffnung aufweist;
wobei die Tür (30) so angepasst ist, dass sie sich zum Öffnen oder Verschließen der Öffnung der Kammer dreht; und wobei sie umfasst:
eine Führungsplatte (70), welche derart an einer Außenfläche der Tür (30) befestigt ist, dass sie um einen vorbestimmten Abstand von der Tür (30) beabstandet ist, wobei die Führungsplatte (70) an einem Ende drehbar mit einer Scharnieranordnung (60) gekoppelt ist,
**dadurch gekennzeichnet, dass** erste Rillen (32) in regelmäßigen Abständen in einer mit der Führungsplatte (70) in Kontakt stehenden Oberfläche der Tür (30) ausgebildet sind und zweite Rillen (72) derart in der Führungsplatte (70) ausgebildet sind, dass sie mit den ersten Rillen (32) in der Tür (30) übereinstimmen, so dass, wenn die Führungsplatte (70) mit der Tür (30) in Kontakt ist, ein Zwischenraum (92) mit einer vorbestimmten Größe zwischen den ersten und zweiten Rillen (32, 72) definiert ist,
ein elastisches Element (90) in den Zwischenraum (92) eingebracht ist, an einem ersten Ende an einer Innenfläche einer jeden der ersten Rillen (32) in der Tür (30) festgehalten ist und an einem zweiten Ende an einer Innenfläche einer jeden der zweiten Rillen (72) in der Führungsplatte (70) festgehalten ist, und
ein Block (86) an der Führungsplatte (70) befestigt ist, der Block (86) so angepasst ist, dass er die Festigkeit der Führungsplatte (70) erhöht, und er ein Loch zum Führen einer Führungsbuchse (84) aufweist.

2. Tür nach Anspruch 1, wobei die Tür (30) in regelmäßigen Abständen Befestigungslöcher (34) mit einer vorbestimmten Tiefe umfasst und die Führungsplatte (70) Durchgangslöcher (74) umfasst, die mit den Befestigungslöchern (34) übereinstimmen, so dass ein Befestigungselement (82) durch ein entsprechendes Durchgangsloch (74) hindurchführbar ist und an einem Ende an dem entsprechenden Befestigungsloch (34) festgehalten wird, wodurch die Befestigung der Führungsplatte (70) an der Tür erfolgt.

3. Tür nach Anspruch 2, wobei eine jede der ersten Rillen (32) in der Tür (30) derart um ein entsprechendes Befestigungsloch herum ausgebildet ist, dass sie eine vorbestimmte Tiefe aufweist, und eine jede der zweiten Rillen (72) in der Führungsplatte (70) derart um ein entsprechendes Durchgangsloch herum ausgebildet ist, dass sie eine vorbestimmte Tiefe aufweist.

4. Tür nach Anspruch 1, wobei das elastische Element (90) eine Schraubenfeder umfasst.

5. Tür nach Anspruch 2, weiterhin umfassend:
eine Führungsbuchse (84) mit einem Loch, durch welches das Befestigungselement (82) hindurchgeführt wird, und die umfasst:
einen ersten Abschnitt, der durch ein entsprechendes Durchgangsloch in der Führungsplatte (70) hindurchführbar ist und um einen vorbestimmten Abstand in den Zwischenraum (92) hineingleitet; und
einen zweiten Abschnitt, der einen größeren Durchmesser als das Durchgangsloch in der Führungsplatte aufweist und dem eine Außenfläche der Führungsplatte (70) als Anschlag dient.

6. Tür nach Anspruch 5, wobei, wenn das Ende des Befestigungselements (82) vollständig an einer jeden der ersten Rillen (32) in der Tür befestigt ist, ein vorbestimmter Spalt zwischen dem zweiten Teil der Führungsbuchse (84) und der Außenfläche der Führungsplatte (70) definiert ist.

7. Tür nach Anspruch 1, wobei eine Basisplatte (40), die in einem Mittelabschnitt eine Öffnung aufweist, an einer Vorderseite der Öffnung der Kammer (20) vorgesehen ist.

8. Tür nach Anspruch 7, wobei die Scharnieranordnung (60) an einem Ende der Basisplatte (40) montiert ist und ein Ende der Führungsplatte (70) drehbar mit der Scharnieranordnung (60) gekoppelt ist.

## Revendications

1. Porte pour une chambre à vide,
la chambre à vide (10) présentant, dans une surface, une ouverture;
la porte (30) étant adaptée de manière à tourner pour ouvrir ou fermer l'ouverture de la chambre; et ladite porte comprenant:
une plaque de guidage (70) qui est fixée à une surface extérieure de la porte (30) de manière à se trouver à une distance prédéfinie de la porte (30), la plaque de guidage (70) étant couplée, à une extrémité, à une disposition de charnière (60) de manière à pouvoir tourner,
**caractérisée en ce que** des premières rainures (32) sont formées à intervalles réguliers dans une surface de la porte (30) se trouvant en contact avec la plaque de guidage (70) et que des deuxièmes rainures (72) sont formées dans la plaque de guidage (70) de manière à coïncider avec les premières rainures (32) ménagées dans la porte (30) de sorte que, lorsque la plaque de guidage (70) est en contact avec la porte (30), un espace (92) avec une grandeur prédéterminée est défini entre les premières et les deuxièmes rainures (32, 72),
un élément élastique (90) est placé dans l'espace (92), est maintenu en place, à une première extrémité, par une surface intérieure de chacune des premières rainures (32) ménagées dans la porte (30) et est maintenu en place, à une deuxième extrémité, par une surface intérieure de chacune des deuxièmes rainures (72) ménagées dans la plaque de guidage (70), et
un bloc (86) est fixé sur la plaque de guidage (70), le bloc (86) est adapté de manière à augmenter la résistance de la plaque de guidage (70) et présente un trou servant à guider une boîte de guidage (84).

2. Porte selon la revendication 1 dans laquelle la porte (30) comprend à intervalles réguliers des trous de fixation (34) avec une profondeur prédéterminée et la plaque de guidage (70) comprend des trous de passage (74) qui coïncident avec les trous de fixation (34) de sorte qu'un élément de fixation (82) passe par un trou de passage correspondant (74) et est maintenu en place, à une extrémité, dans le trou de fixation correspondant (34), fixant ainsi la plaque de guidage (70) sur la porte.

3. Porte selon la revendication 2 dans laquelle chacune des premières rainures (32) ménagées dans la porte (30) est formée autour d'un trou de fixation correspondant de manière à présenter une profondeur prédéterminée et chacune des deuxièmes rainures (72) ménagées dans la plaque de guidage (70) est formée autour d'un trou de passage correspondant de manière à présenter une profondeur prédéterminée.

4. Porte selon la revendication 1 dans laquelle l'élément élastique (90) comprend un ressort hélicoïdal.

5. Porte selon la revendication 2, comprenant en outre:
une boîte de guidage (84) avec un trou à travers lequel passe l'élément de fixation (82) et qui comprend:
une première partie qui passe par un trou de passage correspondant ménagé dans la plaque de guidage (70) et qui est introduite à une distance prédéterminée dans l'espace (92); et
une deuxième partie qui présente un diamètre supérieur au trou de passage ménagé dans la plaque de guidage et à la quelle une surface extérieure de la plaque de guidage (70) sert de butée.

6. Porte selon la revendication 5 dans laquelle, lorsque l'extrémité de l'élément de fixation (82) est complètement fixée sur chacune des premières rainures (32) ménagées dans la porte, un interstice prédéterminé est défini entre la deuxième partie de la boîte de guidage (84) et la surface extérieure de la plaque de guidage (70).

7. Porte selon la revendication 1 dans laquelle une plaque de base (40) qui présente une ouverture dans une partie centrale est prévue sur une face avant de l'ouverture de la chambre (20).

8. Porte selon la revendication 7 dans laquelle la disposition de charnière (60) est montée à une extrémité de la plaque de base (40) et une extrémité de la plaque de guidage (70) est couplée à la disposition de charnière (60) de manière à pouvoir tourner.
